# EUROPEAN PATENT SPECIFICATION

(11) **EP 2 805 163 B1**
(45) Date of publication and mention of the grant of the patent: **08.08.2018**
(21) Application number: 13701301.7
(22) Date of filing: 21.01.2013
(51) Int. Cl.: G01N 33/50

(54) **INDUCED PLURIPOTENT STEM CELL (IPSC)-DERIVED CARDIOMYOCYTE-LIKE CELLS AND USES THEREOF**
AUS INDUZIERTEN PLURIPOTENTEN STAMMZELLEN (IPSC)-GEWONNENE KARDIOMYOZYTEN-ÄHNLICHE ZELLEN UND VERWENDUNGEN DAVON
CELLULES DE TYPE CARDIOMYOCYTE ISSUES DE CELLULES SOUCHES PLURIPOTENTES INDUITES (IPSC) ET LEURS UTILISATIONS

(30) Priority: 20.01.2012 SG 201200530
(43) Date of publication of application: 26.11.2014
(73) Proprietor: Singapore Health Services Pte Ltd, Singapore 168753 (SG)
(72) Inventor: SHIM, Winston, Singapore 169612 (SG); MEHTA, Ashish, Singapore 169612 (SG); WONG, Philip, Singapore 169612 (SG); LIEW, Reginald, Singapore 169857 (SG)
(74) Representative: Bailey, Jennifer Ann
(86) International application number: PCT/SG2013/000029
(87) International publication number: WO 2013/109194

(56) References cited:
- A. MEHTA ET AL: "Pharmacological response of human cardiomyocytes derived from virus-free induced pluripotent stem cells", CARDIOVASCULAR RESEARCH, vol. 91, no. 4, 12 May 2011 (2011-05-12), pages 577-586, XP055047714, ISSN: 0008-6363, DOI: 10.1093/cvr/cvr132
- MEHTA ASHISH ET AL: "Pharmacoelectrophysiology of Viral-Free Induced Pluripotent Stem Cell-Derived Human Cardiomyocytes", TOXICOLOGICAL SCIENCES, ACADEMIC PRESS, SAN DIEGO, FL, US , vol. 131, no. 2 1 February 2013 (2013-02-01), pages 458-469, XP008160596, ISSN: 1096-6080, DOI: 10.1093/TOXSCI/KFS309 Retrieved from the Internet: URL:http://toxsci.oxfordjournals.org/ [retrieved on 2012-01-22]

## Description

### Field of the invention

The present invention relates to in vitro methods for selecting a dosage or dosage range of an agent as a drug candidate customised to an individual subject, comprising the steps of: (i) contacting at least one induced pluripotent stem cell (iPSC)-derived cardiomyocyte-like cell derived from said subject with the agent at more than two different dosages; and (ii) determining the effect of the agent based on changes in at least one electrophysiological property of the iPSC-derived cardiomyocyte-like cell contacted with different dosages of the agent at the different dosages, compared to a control comprising at least one iPSC-derived cardiomyocyte absent the agent to give a dose response curve; and selecting a suitable dosage or dosage range exerting a desired effect on the induced pluripotent stem cell (iPSC)-derived cardiomyocyte like cell(s) from the subject. The present invention further relates to the use of iPSC-derived cardiomyocyte-like cells.

### Background of the invention

Cardiovascular disease is a major cause of morbidity and mortality worldwide and the development of cardiovascular agents requires elaborate safety screening. In addition, cardiotoxicity is an important aspect to consider during the development of both cardiovascular and non-cardiovascular agents. The effect of non-cardiovascular agents on the cardiovascular system can impact on the therapeutic use of such agents. For example, both cardiovascular and non-cardiovascular agents have been withdrawn from the market due to their cardiotoxic effects. Current drug discovery and development programs are inefficient as more than 90% of the lead molecules identified by *in vitro* screening systems fail to become drugs due to safety and efficacy issues in clinical applications. It is therefore desirable to improve on screening methods of agents as drug candidates.

### Summary of the invention

The invention is defined in the claims.

Disclosed is a method for screening an agent as a drug candidate, comprising the steps of:
(i) contacting at least one induced pluripotent stem cell (iPSC)-derived cardiomyocyte-like cell with the agent at different dosages; and
(ii) determining the effect of the agent based on changes in at least one electrophysiological property of the iPSC-derived cardiomyocyte-like cell contacted with different dosages of the agent compared to a control comprising at least one iPSC-derived cardiomyocyte-like cell absent the agent.

Also disclosed is a method for predicting risk of and/or predisposition to cardiac arrhythmia in a subject, comprising:
(i) providing at least one induced pluripotent stem cell (iPSC)-derived cardiomyocyte-like cell derived from the subject;
(ii) contacting the at least one iPSC-derived cardiomyocyte-like cell with a beta-adrenergic agonist;
(iii) further contacting the at least one iPSC-derived cardiomyocyte-like cell from (ii) with a beta-blocker;
(iv) determining a first Beat Rate Variability for the at least one iPSC-derived cardiomyocyte-like cell based on changes in at least one electrophysiological property in the at least one iPSC-derived cardiomyocyte-like cell; and
(v) comparing the first Beat Rate Variability to a second Beat Rate Variability for at least one control.

### Brief description of the figures

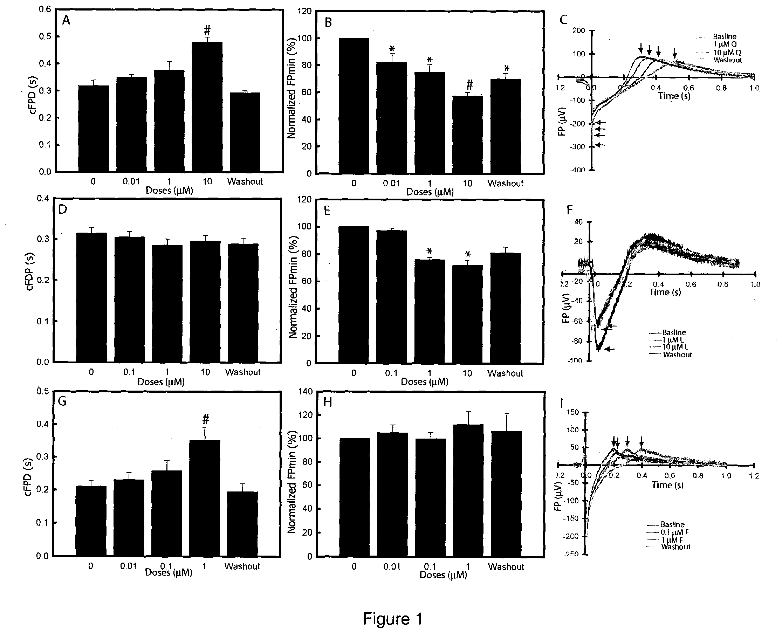

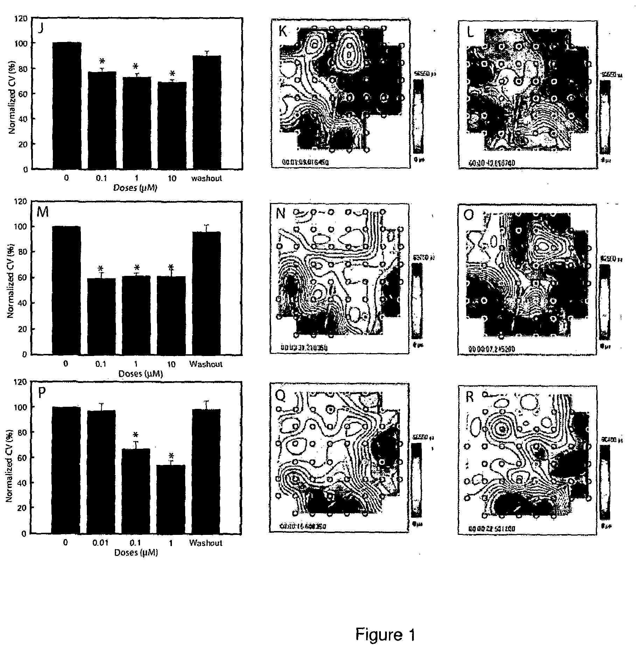

Figure 1 shows the effect of Class I drugs on electrophysiological properties of human induced pluripotent stem cell-derived cardiomyocyte-like cells (hiPSC-CMs). The effects of quinidine (Class la) on cFPD (A), normalized FPmin (B) and multielectrode waveform trace (C); lidocaine (Class Ib) on cFPD (D), normalized FPmin (E) and multielectrode waveform trace (F) and flecainide (Class Ic) on cFPD (G), normalized FPmin (H) and multielectrode waveform trace (I) are shown. The effects of quinidine (J), lidocaine (M) and flecainide (P) on conduction velocity are shown. The heat map images show isochoric lines and the direction of propagation (indicated by arrows) in the presence of 1 µM quinidine (L) compared to control (K), 10 µM lidocaine (O) compared to control (N) and 1 µM flecainide (R) compared to control.
Figure 2 shows the effect of beta-blockers on electrophysiological properties of hiPSC-CMs. The effects of nadolol on cFPD (A) and beating frequency (B, C) and propranolol on cFPD (D) and beating frequency (E, F) are shown.
Figure 3 shows the effects of K⁺ channel blockers on electrophysiological properties of hiPSC-CMs. The effects of sotalol (Class II and III) on cFPD (A), multielectrode waveform trace (B); and amiodarone (Class III with Class I, II and IV activities) on cFPD (C), multielectrode waverform trace (D) are shown.
Figure 4 shows the effects of verapamil (Class IV) on the cFPD (A), normalized FPmin (B) and multielectrode waveform trace (C).
Figure 5 shows the non-linear regression analysis of dose dependent response of various drugs on cFPDs. The regression analyses are overlaid with the human unbound therapeutic concentration (ETPC unbound) range for various different agents.
Figure 6 shows the effect of different doses of quinidine (A), flecainide (B), propranolol (C), sotalol (D), amiodarone (E) and verapamil (F) on the beating rhythm following drug treatments.

### Definitions

The term "cardiomyocyte-like cell" is intended to mean a cell sharing features with a cardiomyocyte. Cardiomyocyte-like cells are further defined by morphological characteristics as well as by specific marker characteristics. As induced pluripotent stem cell-derived cardiomyocyte-like cells share similar characteristics (including marker and electrophysiological characteristics) with cardiomyocytes, induced pluripotent derived cardiomyocyte-like cells may be used interchangeably with induced pluripotent stem cell-derived cardiomyocytes.

Cardiovascular agent refers to agents that have potential to ameliorate, control, eliminate, prevent, reduce and/or treat a wide variety of disorders related to the heart and/or circulation.

Conduction velocity refers to the speed at which cardiomyocytes propagate an electrical impulse across cardiac tissue or syncytium.

An "embryoid body" refers to an aggregate of cells derived from pluripotent cells, where cell aggregation can be initiated by any method that prevents the cells from adhering to a surface to form typical colony growth.

FPD or field potential duration corresponds to the action potential duration, which can be correlated to a QT interval in an electrocardiogram. It is measured from minimum of the Na+ peak to the maximum/minimum of the I_{Kr} current peak.

FPmin corresponds to maximal negative amplitude of field potential that depends on critically on the current through voltage dependent Na+-channels

As used herein, the term "induced pluripotent stem cells" refers to a pluripotent stem cell derived from a somatic cell (e.g. an adult somatic cell). Induced pluripotent stem cells are similar to embryonic stem cells in their differentiation abilities to form any adult cell types, but are not derived from an embryo.

As used herein, the term "pluripotent" refers to the potential of a stem cell to make any differentiated cell type of an organism. Pluripotent stem cells can give rise to any fetal or adult cell type. However, alone they cannot develop into a fetal or adult organism because they lack the potential to contribute to extraembryonic tissue, such as the placenta.

As used herein, the terms "Heart Rate Variability" (HRV) and "Beat Rate Variability" (BRV) are used interchangeably to refer to the physiological phenomenon of variation in the time interval between periodic changes in at least one electrophysiological property, for example between heartbeats in a beating heart, or the beat-to-beat interval. When measured on a beating heart, Beat Rate Variability refers to Heart Rate Variability. BRV may be measured using various methods including ECG, blood pressure, ballistocardiograms, the pulse wave signal derived from a photoplethysmograph, the power spectral density of beat-to-beat or RR intervals, field potential duration (FPD), minimum field potential (FPmin), conduction velocity, beating frequency and/or regularity of beating rhythm, and other methods known in the art.

Cardiac arrhythmia (also commonly known as dysrhythmia or irregular heartbeat) is associated to a heartbeat that beats either too fast or too slow. The heartbeat may also beat regularly or irregularly. Cardiac arrhythmia comes from any of a large and heterogeneous group of conditions in which there is abnormal electrical activity in the heart.

"Risk of" and/or "predisposition to" cardiac arrhythmia in a subject, as used in this specification, refers to the probability that the subject has, or will develop, cardiac arrhythmia in the future or near future. For example, a subject may consider various options for treatment or take up various preventative measures if a subject predicts risk of cardiac arrhythmia as far in advance as possible. Risk of cardiac arrhythmia in a subject may be due to genetic and non-genetic factors, for example a family history of cardiac arrhythmia, scarring of heart tissue (such as from a heart attack), electrolyte imbalances in blood, coronary artery disease, or other risk factors. Accordingly, severity of this risk in a subject is determined in comparison to the probability of cardiac arrhythmia in control groups, for example a group of normal individuals (i.e. individuals without cardiac arrhythmia risk factors) and/or a group of individuals with cardiac arrhythmia or having risk factors for cardiac arrhythmia.

### Detailed description of the invention

Disclosed is a method for screening an agent as a drug candidate, comprising the steps of:
(i) contacting at least one induced pluripotent stem cell (iPSC)-derived cardiomyocyte-like cell with the agent at different dosages; and
(ii) determining the effect of the agent based on changes in at least one electrophysiological property of the iPSC-derived cardiomyocyte-like cell contacted with different dosages of the agent compared to a control comprising at least one iPSC-derived cardiomyocyte absent the agent. The electrophysiological property includes the field potential duration (FPD), minimum field potential (FPmin), conduction velocity, beating frequency and/or regularity of beating rhythm and may be measured with a multielectrode array or patch-clamp. The more sophisticated planar patch-clamp systems which are potentially suited for high throughput screening may also be used.

Accordingly, the method may be used to determine if an agent causes an increase, decrease or no changes in the field potential duration (FPD) compared to the control. The method may also be used to determine if an agent causes an increase, decease or no changes in the minimum field potential (FPmin). The method may also be used to determine if an agent causes an increase, decrease or no changes in the conduction velocity compared to the control. The method may also be used to determine if an agent causes an increase, decrease or no changes in the beating frequency compared to the control. The method may also be used to determine if an agent causes a change or no changes in regularity of beating rhythm compared to the control. The method may thus be used for screening a drug candidate. In particular, the method may be used to screen for an agent exerting an effect on cardiomyocytes. For example, the method may be used to screen for an agent exerting any one of the following effects:
(i) increasing the field potential duration (FPD) compared to the control.
(ii) decreasing the field potential duration (FPD) compared to the control.
(iii) increasing the minimum field potential (FPmin) compared to the control.
(iv) decreasing the minimum field potential (FPmin) compared to the control.
(v) increasing the beating frequency compared to the control.
(vi) decreasing the beating frequency compared to the control
(vii) increasing conduction velocity compared to the control
(viii) decreasing conduction velocity compared to the control; or
(ix) disrupting the regularity of rhythmic waveform compared to the control.

Correspondingly, the control refers to the relevant electrophysiological property of the iPSC-cardiomyocyte-like cell absent the agent.

Further, depending on the effect exhibited, the agent may be useful for ameliorating, controlling, eliminating, preventing, reducing and/or treating a cardiac condition and may be further tested. Accordingly, the method may screen for a cardiovascular agent, an agent affecting cardiac function and/or an agent for ameliorating, controlling, eliminating, preventing, reducing and/or reducing a cardiac condition. In particular, the method may be for screening anti-arrhythmic agents.

The present method may also be used for assessing the cardiotoxicty of the agent. For example, an agent may be considered cardiotoxic if the agent excessively exerts an effect on at least one iPSC-derived cardiomyocyte compared to the control. An agent may also be considered cardiotoxic if it increases or decreases at least one electrophysiological property to such an extent that it cannot be considered safe for use. For example, an agent that increases the field potential duration (FPD), minimum field potential (Fpmin), conduction velocity, and/or the beating frequency excessively is unlikely to be safe. Alternatively, an agent that decreases the field potential duration (FPD), minimum field potential, (Fpmin), conduction velocity and/or the beating frequency, excessively (for example, almost to a flat line) is unlikely to be safe. Further, an agent may also be considered cardiotoxic if it causes a change in the regularity of beating rhythm, for example an irregular beating rhythm. The cardiotoxicity of agents with therapeutic applications other than cardiovascular therapy (i.e. non-cardiovascular agents) may also be assessed.

Further, the method may be used for selecting a dosage and/or dosage range of the agent. The induced pluripotent stem cell-derived cardiomyocyte-like cell is a useful model to select for suitable dosage and/or dosage ranges. For example, the effect of various dosages of the agent is compared with said control and a suitable dosage or dosage range of the agent capable of exerting said effect may be selected. In particular, a suitable dosage and/or dosage range would be one exerts an effect likely to be therapeutically effective with minimal cardiotoxicity.

Induced pluripotent stem cells (iPSC) may be generated from adult somatic cells by any method, including but not limited to reprogramming to the embryonic state by viral or non-viral based methods. These iPSCs resemble embryonic stem cells in self renewal capacities and differentiation potential to various cell types including cardiomyocytes. For example, iPSCs may further be differentiated into cardiomycocyte-like cells by any method. Such induced pluripotent stem cell (iPSC)-derived cardiomyocyte-like cells (iPSC-CMs) were found to be a useful model for screening agents as drug candidates .According to a particular aspect, human induced pluripotent stem cells (hiPSCs) may be used to derive human induced pluripotent stem cell-derived cardiomyocyte-like cells (hiPSC-CMs).

The present disclosure is suitable for screening agents customised to an individual subject. Accordingly, cardiomyocyte-like cells derived from Induced pluripotent stem cells from said individual subject may be used in screening an agent for said individual subject. "Personalised medicine" is desired in the field of therapeutics and the present invention may be used to test variations in therapeutic effects of an agent for an individual.

Any suitable method may be used for preparing cardiomyocyte-like cells from induced pluripotent stem cell. For example, induced pluripotent stem cells may be generated by a process of reprogramming of somatic cells isolated from normal or diseased subjects. These hiPSC are then differentiated in vitro to cardiomyocytes. Accordingly, the induced pluripotent stem cell-derived cardiomyocyte-like cell(s) are already isolated (from the human or animal body). Accordingly, the method according to any aspect of the invention is performed *in vitro.*

The present disclosure also relates to Induced pluripotent stem cell (iPSC) derived cardiomyocyte-like cell(s) for use in screening an agent as a drug candidate.

Further, the disclosure also relates to Induced pluripotent stem cell (iPSC)-derived cardiomyocyte-like cell(s) for use in a method according to any aspect disclosed herein. The disclosure also includes a kit comprising these induced pluripotent stem cell (iPSC)-derived cardiomyocyte-like cell(s).

Moreover, the reduced BRV of hiPSC-CMs from individual with genetically predisposed arrhythmia further supported the feasibility of using such hiPSC-CMs to prognosticate cardiac arrhythmia. Accordingly, in a second aspect the present disclosure provides a method for predicting risk of and/or predisposition to cardiac arrhythmia in a subject, comprising:
(i) providing at least one induced pluripotent stem cell (iPSC)-derived cardiomyocyte-like cell derived from the subject;
(ii) contacting the at least one iPSC-derived cardiomyocyte-like cell with a beta-adrenergic agonist;
(iii) further contacting the at least one iPSC-derived cardiomyocyte-like cell from (ii) with a beta-blocker;
(iv) determining a first Beat Rate Variability (BRV) for the at least one iPSC-derived cardiomyocyte-like cell based on changes in at least one electrophysiological property in the at least one iPSC-derived cardiomyocyte-like cell; and
(v) comparing the first BRV to a second BRV for at least one control.

Beat Rate Variability may be determined using data from the above measurements and using any suitable algorithms and/or software. If the first Beat Rate Variability is lower than the second Beat Rate Variability, this may be indicative of risk of and/or predisposition to cardiac arrhythmia in the subject as compared to a normal individual.

In (v) of the above method, the first Beat Rate Variability may be compared to any suitable control or set of controls in order to determine the severity of the risk and/or predisposition to cardiac arrhythmia in the subject. For example, at least one control comprising at least one iPSC-derived cardiomyocyte-like cell derived from an individual may be used. The individual may be a normal individual lacking risk factors for cardiac arrhythmia, or an individual known to have risk factors or known to have cardiac arrhythmia. Other controls may also be used, for example comprising cardiomyocyte(s) from at least one of the abovementioned individuals. HRV measurements of the above individuals may also serve as suitable controls, i.e. the first BRV may be compared to at least one HRV from at least one of the abovementioned individuals. While generally accepted threshold values for declaring a safe boundary indicating normal HRV may not be well established at this point, such values if established in the future may be suitable as controls as well.

When the at least one control comprises at least one iPSC-derived cardiomyocyte-like cell derived from a normal individual, if the first Beat Rate Variability is lower than the second Beat Rate Variability, this may be indicative of risk of and/or predisposition to cardiac arrhythmia in the subject as compared to a normal individual.

In the above method, (iv) may comprise measuring at least one electrophysiological property in the at least one iPSC-derived cardiomyocyte-like cell, the at least one electrophysiological property comprising the power spectral density of beat-to-beat or RR intervals, ECG, blood pressure, ballistocardiograms, the pulse wave signal derived from a photoplethysmograph, field potential duration (FPD), minimum field potential (FPmin), conduction velocity, beating frequency and/or regularity of beating rhythm. The method (at (iv)) may comprise measuring the power spectral density of beat-to-beat or RR intervals in the at least one iPSC-derived cardiomyocyte-like cell. The method (at (iv)) may comprise measuring at least one electrophysiological property in the iPSC-derived cardiomyocyte-like cell(s) after (ii) and after (iii). The iPSC-derived cardiomyocyte-like cell(s) of the method may be derived from human iPSC. Any suitable beta-adrenergic agonist may be used in the method, for example isoprenaline. Any suitable beta-blocker may be used in the method, for example propranolol, atenolol.

Further, the disclosure also relates to Induced pluripotent stem cell (iPSC)-derived cardiomyocyte-like cell(s) for use in a method for predicting risk of and/or predisposition to cardiac arrhythmia in a subject according to any aspect of the invention. The disclosure also a kit comprising these induced pluripotent stem cell (iPSC)-derived cardiomyocyte-like cell(s).

It is understood that the method for predicting risk of and/or predisposition to cardiac arrhythmia in a subject is an *in vitro* method.

Having now generally described the invention, the same will be more readily understood through reference to the following examples which are provided by way of illustration, and are not intended to be limiting of the present invention.

### EXAMPLES

In a proof of principle study, the effects of various agents including four classes of anti-arrhythmic drugs classified according to the Vaughan Williams system and beta blockers were assessed using hiPSC-CMs as an *in vitro* model.

The US Food and Drug Administration (FDA) defined prolongation of QT interval on an ECG as a major drug safety issue. Consistently, assessing risk for QT interval prolongation is part of the standard preclinical evaluation of New Chemical Entities (NCEs) as defined by the International Conference of Harmonization (ICH) Expert Working Group in topic S7B for all drugs in development. QT prolongation tracks cardiac repolarization reserve and is an indicator for increased risk of torsade de pointes (TdP), a life threatening polymorphic ventricular tachycardia. Drug-induced prolongation of QT-interval is often evoked by affecting the rapid potassium current I_{Kr} through disruption of hERG ion channel activity. While evaluations of QT interval changes may be a prime requisite, not all drugs may directly interfere with hERG channels. In addition to potassium channels, the electrical activity of a cardiomyocyte is largely dependent on voltage-gated sodium and calcium channels. Whereas activation of sodium channels initiates an action potential, inward Ca²⁺ currents through (L-type) voltage-gated calcium channels sustain membrane depolarization, but are counterbalanced by outward potassium currents in the repolarization phase to complete a cardiac cycle. Thus, it is also important to evaluate additional phases along with QT interval to generate a comprehensive electrical profile throughout the cardiac cycle.

In this study, various electrophysiological properties of hiPSC-CMs when exposed to the different agents were studied.

### Materials and Methods

### Drugs

The following anti-arrhythmic drugs were studied:
Quinidine (Class la)
Lidocaine (Class Ib)
Flecainide (Class Ic)
Sotalol (Class II and III)
Amiodarone (Class III with I, II and IV activities)
Verapamil (Class IV)
Nadolol (Class II)
Atenolol (Class II)
Propranolol (Class II)

### Cell lines and culture

An episomal based method was utilized to reprogram fibroblast as reported previously (Mehta *et al.,* 2011). Human iPSC lines [MSnviPSNF2 (C2), MSnviPSNF3 (C3)] were maintained on 1% matrigel (BD Biosciences, CA, USA) and grown in chemically defined mTeSR1 medium (Stem Cell Technologies, VA, Canada). Care was taken to manually remove the differentiated areas (<10%) with the help of a scraper before passaging. Cells were washed with DMEM-F12 (Invitrogen, USA) and incubated with 1 mg/ml dispase (Sigma, USA) for 5-7 minutes. Cells were then washed with DMEM-F12 and colonies scrapped from the culture dishes. Controlled tituration of the colonies was performed to get small clumps of approximately 50-70 µm in size for replating on new matrigel coated dishes (1:6 split ratio). Cells were fed daily with fresh mTeSR1 medium (StemCell Technologies Inc, VA, Canada) and passaged every 5-6 days and maintained at 37 °C under 5% CO2 in air at 95% humidity.

### EB formation and cardiomvocvte differentiation

For example, cardiomyocyte differentiation was performed following EB formation as reported previously (Mehta *et al.,* 2011). Briefly, hiPSCs colonies were dispersed into small clumps and placed in low adhesion culture dishes in EB medium as described previously (Mehta et al., 2010) with 5 µM of SB203580 (Calbiochem, USA) for 8 days. Subsequently, EBs were plated on 0.1% gelatin coated dishes in EB medium without SB203580. Beating areas were typically observed around day 10-12 from EB formation. Beating areas were manually cut after day 21 of differentiation and utilized for various experiments.

Viral free hiPSC-derived cardiomyocytes express hallmark cardiac-specific structural and sarcomeric proteins (actinin, troponins, MYH7, and MLC2v, titin), gap-junction proteins like connexin 43, ion channel proteins [Cav1.3, encoding the α1D subunit of the L-type calcium channel; KCNH2 (hERG), mediating the rapid delayed rectifier potassium current (I_{Kr}); and the hyperpolarization-activated cyclic nucleotide-gated potassium channel (HCN2), responsible for the If pacemaker current, SERCA and Na-Ca exchanger] based on gene and protein expressions (Mehta *et al.,* 2011)

### Multielectrode Array (MEA) Electrophysiology

To characterize the electrophysiological properties of the hiPSC-CMs, a multielectrode array (MEA) recording system (Multichannel Systems, Reutlingen, Germany) was used. The contracting areas were microdissected and plated on gelatin coated MEA plates. The MEA system allows simultaneous recordings from 60 titanium nitride electrodes (30 µm) at high spatial (200 µm) and temporal (15 kHz) resolutions. To assess the effects of different drugs on the electrophysiological properties, the stock drugs in DMSO were diluted in medium (2 mL). All drugs utilized in the study were purchased from Sigma, USA. Extracellular recording were performed for 180-300 seconds at baseline and at 5 minutes after drug application. The local activation maps were generated using Cardio2D software (Multichannel Systems, Reutlingen, Germany). The recorded electrograms were also used to determine the local field potential (FP) duration (FPD). FPD measurements were normalized (corrected FPD [cFPD]) to the beating rate of the contracting areas with the Bazzet correction formula: cFPD_FPD/√(RR interval) as described previously (Zwi *et al.,* 2009). Conduction velocity was also determined as described in Zwi *et al.,* (2009).

### Beat Rate Variability Analysis in beta-adrenergic response

To study the autonomic modulations of beating period of the contracting EBs, power spectral density of beat-to-beat or RR intervals based on 5-min recordings from the MEA were used to derive the Total Power (TP), very low frequency power (VLF), low frequency power (LF), high frequency power (HF), LF (normalized unit), HF (normalized unit) and ratio of LF/HF of spectral of beat rate variability using software such as HRV Toolkit (physionet.org/tutorials/hrv-toolkit/, Harvard Medical School) or LabVIEW Biomedical Toolkit for heart rate variability. Contracting EBs from normal healthy donor or individual with genetic predisposition to cardiac arrhythmia was exposed to 0.1 uM of isoprenaline and subsequently suppressed with 1 uM beta-blocker (propranolol or atenolol) to study sympathvagal balance. The data were obtained using iPSC-derived cardiomyocyte-like cells from an individual with genetic pre-disposition to cardiac arrhythmia (n = 4; referring to 4 regional areas within an EB), and iPSC-derived cardiomyocyte-like cells from a normal individual without genetic predisposition to cardiac arrhythmia (n = 10).

### Statistical Analysis

Comparisons at each time point were conducted using analysis of variance (ANOVA), and all data are presented as mean values ± s.e.m of three independent experiments. Differences were considered statistically significant at p ≤ 0.05. Corrected field potential duration curves were fitted using the one phase decay algorithm from GraphPad Prism software. 95% confidence intervals are plotted as dashed lines.

### Results

### Anti-arrhythmogenic drug induced electrical alterations in hiPSC-CMs

### a) Effects of sodium (Na⁺) channel blockers on hiPSC-CMs

The effects of clinical drugs that affect Na⁺ channels on hiPSC-CMs were evaluated. Quinidine (Class la) which primarily blocks the fast inward sodium current (I_{Na}) but with potassium channel blocking abilities, demonstrated a significant 55 % cFPD prolongation at 10 µM dose (Figure 1A, C). Furthermore, quinidine similarly demonstrated a 62% cFPD prolongation in CMs derived from C2 line (cFPD, baseline vs 10 µM Q: 0.31±0.03s vs 0.50±0.05s, n=3, p<0.05). Quinidine demonstrated a dose dependent shortening of FPmin, maximum shortening of 37 % was observed at a 10 µM concentration in C3 cell line derived CMs (Fig 1B-C) while 55 % FPmin shortening was observed with C2 cell line derived CMs (FPmin (normalized), baseline vs 10 µM Q: 100±0.0%s vs 45±3.5 %, n=3, p<0.05).

Lidocaine (Class Ib), which blocks Na+ influx through voltage-gated Na+ channels, did not demonstrate any significant changes in cFPD (Figure 1D, F) with either cell line derived CMs. However, dose-dependent treatment with lidocaine resulted in FPmin shortening with its maximal effects at a 10 µM dosage (Figure 1E-F).

Flecainide (Class Ic), which blocks sodium current with slow kinetics, caused 1.7 fold increase in cFPDs at 1µM concentrations (Figure 1G, I). Similar results were observed with C2 derived CMs (cFPD, baseline vs 1 µM Q: 0.32±0.04s vs 0.55±0.06s, n=3, p<0.05). However, both cell line derived CMs did not show any significant change in FPmin following flecainide treatments (Figure 1H-I). Importantly, those drug-induced changes were reversible following drug removal (washout) (Fig 1).

### b) Effects of beta-blockers on hiPSC-CMs

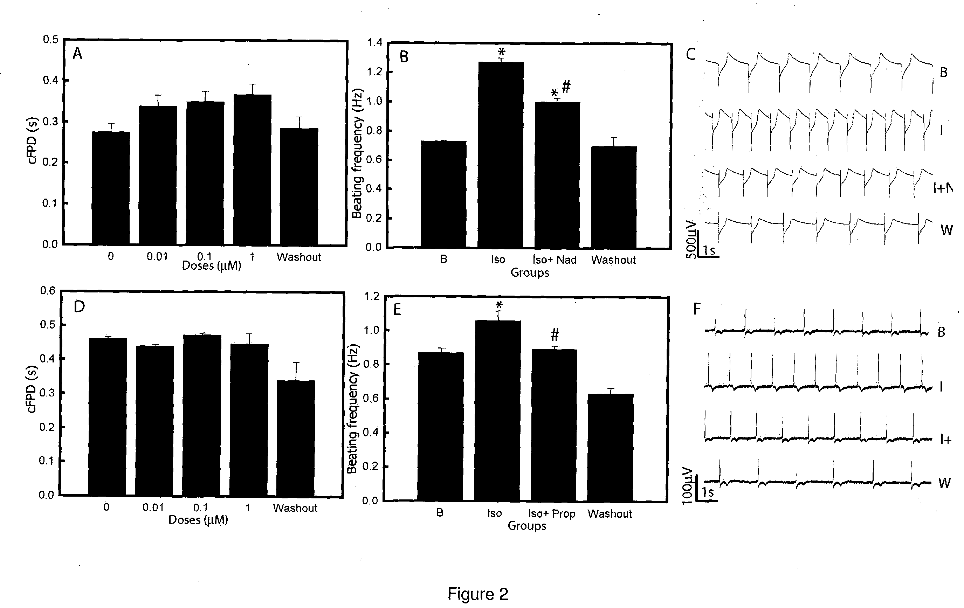

The effects of two beta-blockers, nadolol and propranolol on the hIPSC-CM clusters were evaluated. Both nadolol and propranolol did not have any significant effects on cFPD from both cell lines (Figure 2A, D) and also FPmin. However, clusters initially treated with beta-adrenergic agonist, isoprenaline, demonstrated a significant increase in beating frequencies that could be reversed by both the beta-blockers (Figure 2B-C, E-F). Furthermore, isoprenaline (0.1 µM) treatment reduced cFPD by 20% (cFPD (normalized): baseline vs Iso: 100±0.0 % vs 81.2 ±2.4 %, n=3, p<0.05). This reduced cFPD could be reversed effectively with nadolol (cFPD (normalized), Iso vs nad: 81.2 ±2.4 vs % 92.3 ± 1.5 %, n=3, p<0.05) as well as propranolol (cFPD (normalized), Iso vs pro: 79.6 ±3.4 vs % 98.3 ± 4.5 %, n=3, p<0.05).

### c) Effects of potassium (K⁺) channel blockers on hiPSC-CMs

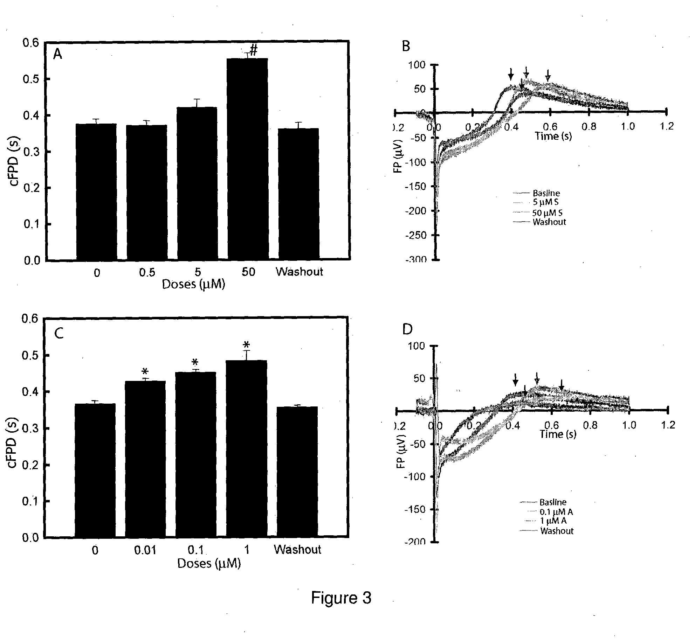

Sotalol (Class II and III) and amiodarone (Class III with I, II and IV activities), both demonstrated prolongation of cFPDs (Figure 3A, C). While sotalol at 50 µM concentration demonstrated a 65% prolongation (Figure 3A-B), amiodarone (1 µM) showed a 37 % prolongation (Fig 3C-D). Similar results were also observed with C2 derived CM with amiodarone (cFPD: baseline vs 1 µM A: 0.26±0.009s vs 0.36 ± 0.01s, n=3, p<0.05). While sotalol treatment did not show any significant change in FPmin levels (data not shown), amiodarone demonstrated differential effects of FPmin shortening with both cell lines. While C3 derived CMs showed only 10 % reduction (FPmin (normalized values) baseline vs 1 µM A: 100±0 vs 90±2.7, n=3; ns), C2 derived CMs demonstrated 25% shortening (FPmin (normalized values) baseline vs 1 µM A: 100±0 vs 73.6±3.8, n=3; p<0.05).

### d) Effects on calcium (Ca²⁺) channel blockers on hiPSC-CMs

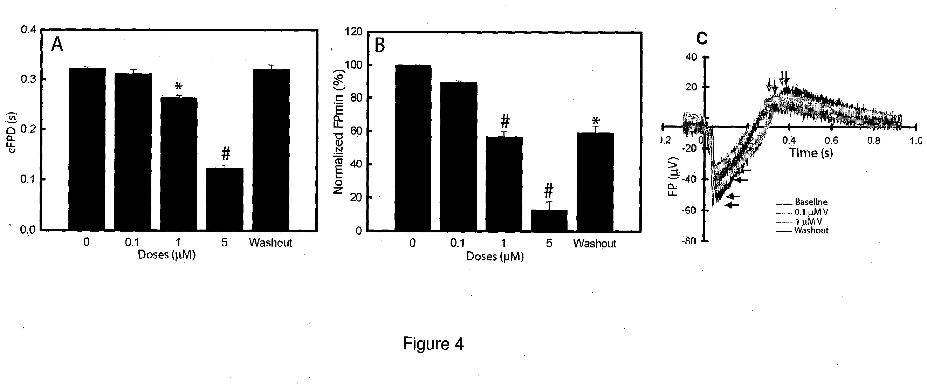

The effect of verapamil (Class IV), a calcium channel blocker and also a potent hERG blocker, was evaluated. cFPD reduced by 60% following 5 µM verapamil treatment (Figure 4A,C). Prolonged exposure to verapamil at 5 µM concentration resulted in beating arrest. Furthermore, verapamil also reduced FPmin by 40% and 80 % at 1 µM and 5 µM concentrations, respectively (Figure 4B-C).

### Drug induced cFPD changes and clinical relevance

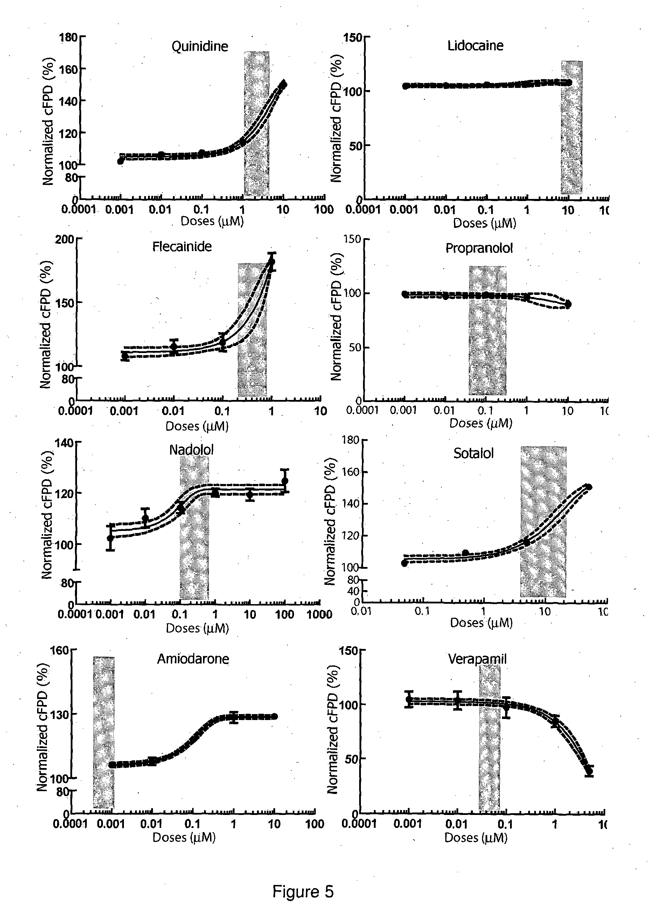

Alterations in QT interval is one of the critical criteria for drug evaluations on cardiac tissue and the local field potential duration (FPD) of cardiomyocytes reflects the local QT interval (Zwi *et al.,* 2009). Thus, non-linear dose dependent (range 10⁻⁹-10⁻⁴ M) regression analysis utilizing one phase decay algorithms on normalized cFPD was performed. These regression analyses were overlaid with human estimated unbound therapeutic plasma concentration (ETPC unbound) ranges (Figure 5) Interestingly, cFPD prolongation effects for quinidine, flecainide and sotalol were maximally observed within the human ETPC range. However, amiodarone only demonstrated cFPD prolongations beyond the ETPC range. On the other hand, lidocaine, propranolol and verapamil did not show any change in cFPDs within the ETPC range. Furthermore, verapamil only show demonstratable cFPD shortening far beyond the ETPC range.

### Drug-induced arrhythmia

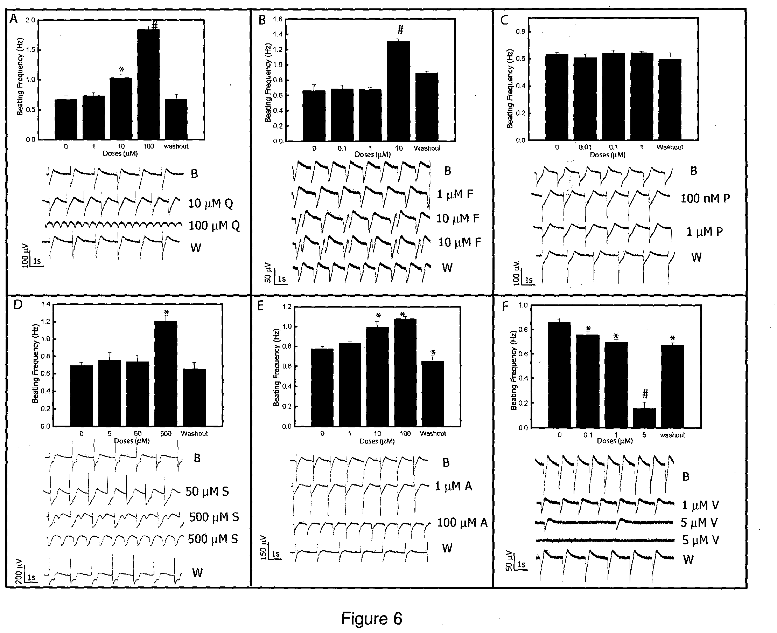

The different agents were also evaluated to determine if these agents (drugs) could induce arrhythmia in hiPSC-CMs at supraphysiological ranges. Human iPSC-CMs were treated with higher (10-100 µM) doses to study their effects on regularity of beating rhythm. Quinidine enhanced beating frequencies by 3-fold at 100 µM (BF: baseline vs 100 µM Q: 0.66±0.06 Hz vs 1.84±0.08 Hz, n=3, p<0.05). Furthermore, there was a significant alteration in the waveforms at 100 µM with resemblance to tachycardia (Figure 6A). These sustained tachycardia-like waveforms were followed by contraction arrest eventually. Similar results were observed with C2 derived CM by at 50 µM concentrations (data not shown). Similarly, flecainide (10 µM) caused 2-fold increase in beating frequencies (Figure 6B), but generated waveforms similar to bigeminy (initially, first 3-5 min) followed by trigeminy-like waveforms (later, >5min). Nevertheless, all waveforms returned towards sinus rhythm following drug removal, though at different time intervals. Unlike Na+ channel blockers, beta blockers (propranolol) did not show any major effect on beating frequencies (Figure 6C). Sotalol increased beating frequencies by 1.8-fold at 500 µM. Initial exposure to 500 µM resulted in early after depolarizations (EADs), which on continuous exposure resulted in sustained arrhythmia (Figure 6D). In contrast, though amiodarone increased beating frequencies at 10-100 µM, the waveform was not observed to be altered significantly. (Figure 6E). In contrary to other drugs, verapamil (5 µM) significantly reduced beating frequencies that subsequently resulted in sinus arrest that recovered upon drug withdrawal (Figure 6F).

### Conduction velocity

The effects of sodium (Na⁺) channel blockers (quinidine, lidocaine and flecainide) at different doses (range 0.01-10 µM) on conduction velocity were studied. Significant and differential reduction of conduction velocity was observed with the sodium channel blockers examined. While quinidine reduced conduction velocity by 20-25% over the three tested doses (Figure 1 J-L, lidocaine demonstrated a 40% reduction in conduction velocity in all three doses tested (Fig 1M-O). Interestingly, flecainide did not show any significant reduction of conduction velocity at the lowest does (0.01 µM) but demonstrated a significant reduction of 45% in conduction velocity at 1 µM (Figure 1 P-R). The reduction in conduction velocity could also be observed from heat map images of the different sodium channel blockers .Each isochoric line in the heat map images shows a propagated distance in 5 milliseconds. Drug treated isochoric lines [quinidine (L); lidocaine (O) flecainide (R)] are closer to each other compared to the respective controls (k, N and Q), indicative of slower conduction velocity for the treated cells. The heat images also showed the direction of propagation, as indicated by the arrows.

### Beat Rate Variability

The spectral analysis indicated that contracting EBs derived from individual with genetic disposition to arrhythmia has a lower beat rate variability (BRV) in comparison to contracting EBs derived from normal healthy donor whereby TP with isoprenaline: 0.464 ± 0.047 ms² vs. 0.645 ± 0.001 ms², p< 0.005 and TP with isoprenaline + beta-blocker: 0.345 ± 0.052 ms² vs. 0.494 ± 0.010 ms², p<0.01. VLF with isoprenaline: 0.341 ± 0.167 ms² vs. 0.578 ± 0.002 ms², p=NS and VLF with isoprenaline + beta-blocker: 0.227 ± 0.078 ms² vs. 0.403 ± 0.020 ms², p<0.05. LF with isoprenaline: 0.058 ± 0.043 ms² vs. 0.050 ± 0.001 ms², p=NS and LF with isoprenaline + beta-blocker: 0.072 ± 0.078 ms² vs. 0.068 ± 0.018 ms², p=NS. HF with isoprenaline: 0.064 ± 0.095 ms² vs. 0.017 ± 0.002 ms², p=NS and HF with isoprenaline + beta-blocker: 0.045 ± 0.043 ms² vs. 0.022 ± 0.012 ms², p=NS. LF (normalized unit) with isoprenaline: 61.136 ± 16.674 % vs. 74.505 ± 2.380 %, p=NS and LF (normalized unit) with isoprenaline + beta-blocker: 63.009 ± 11.372 % vs. 76.711 ± 5.109 %, p<0.01. HF (normalized unit) with isoprenaline: 38.864 ± 16.674 % vs. 25.494 ± 2.381 %, p=NS and HF (normalized unit) with isoprenaline + beta-blocker: 36.990 ± 11.372 % vs. 23.289 ± 5.109 %, p<0.01. LF/HF ratio with isoprenaline: 1.897 ± 1.052 vs. 2.949 ± 0.326, p=NS and LF/HF ratio with isoprenaline + beta-blocker: 1.866 ± 0.711 vs. 3.459 ± 0.847, p<0.01. Such overall reduced BRV in contracting EBs from individual with genetic disposition to arrhythmia is consistent with clinical observations of association of increased arrhythmia and sudden cardiac death with reduced HRV (Lombardi et al 2001).

### Discussion

Three subclasses of Class I anti-arrhythmic drugs, quinidine (Class la), lidocaine (Class Ib) and flecainide (Class Ic) on the electrophysiological properties of hiPSC-CMs were evaluated. Quinindine and lidocaine both demonstrated significant reduced FPmin values that reflect their expected effects on Na channels. However, quinidine also demonstrated significant prolongation of cFPDs at doses that were within the ETPC unbound range (0.9-32 µM), whereas lidocaine did not cause any cFPD prolongation. The delayed repolarization effect on cFPD prolongation with quinidine may be attributed to its highly complex mode of action that involves multiple channels, including hERG. Consistently, concentration-dose inhibition curves for hERG/I_{Kr}, APD90 and QT prolongation by quinidine were known to be tightly correlated (Redfern *et al.,* 2003). Furthermore, quinidine is utilized clinically to modulate QT in rhythm management, though with considerable proarrhythmic risks. Furthermore, the results demonstrated tachycardia-like arrhythmia at high doses demonstrating considerable risk associated with quinidine. In contrast, amiodarone which has excellent safety profile clinically, such proarrhythmic risk was not observed, even at supraphysiologically elevated dosage.

Interestingly, flecainide (Class Ic) was observed to cause a significant increase in cFPDs within the ETPC unbound range (500-900 µM) and at higher doses, arrhythmias with bigeminy/trigeminy-like waveforms were observed (10 µM). Although, flecainide is widely used to supress atrial fibrillation, it is associated with an increased risk of ventricular proarrhythmia. This could be due to the putative differential effects of flecainide on atrial and ventricular IK1, could account for the selective prolongation of cFPDs and arrhythmia. Initial patch clamp studies have shown that these hiPSC-CM clusters have about 75% ventricular cells and about 20-25% atrial cells The presence of artial population within these clusters may contribute to the initial cFPD prolongation and later at higher doses (1-10 µM) would block hERG channels (IC50 >3µM, Redfern et *al.,* 2003) in both cell types leading to arrhythmias.

Consistent with earlier published reports (Yookoo *et al.,* 2009), both nadolol and propranolol significantly suppressed beating frequency in the presence of isoprenaline. However, in contrary to clinical observations, modulation of beating rates was not apparent when either beta-blocker was applied alone. Furthermore, there was no significant cFPD prolongation within the ETPC unbound range with either of the drug (nadolol: 0.18-0.80⁻ µM, propranodolol: 0.06-0.45 µM).

Although both sotalol and amiodarone increased cFPDs, sotalol had no effect on FPmin (Na currents), while only amiodarone showed changes in FPmin levels. Furthermore, cFPD prolongation with sotalol was maximally observed within the ETPC unbound range (1.8-14 µM), amiodarone caused cFPD prolongations only at a much higher levels than ETPC range (0.3-0.5 nM). These results correlated well with the existing clinical observations whereby amiodarone has an excellent safety profile while sotalol is well-known to lengthen repolarization and refractoriness. Consistently, sotalol at higher dose (500 µM) induced early afterdepolarizations (EADs). while amiodarone maintained regular rhythmic contractions even at highest concentration tested. Due to its broad spectrum activities across different ion channels, there has been considerable variability in the reported action of amiodarone in literature. For example, amiodarone has been reported to prolong, shorten or ineffective in APD modifications (reviewed by Kodama *et al.,* (1997). Similarly, some variations in its ability to block Na currents were reported (reviewed by Kodama et al., 1997). cFPD prolongations along with differential FPmin effects on the cardiomycytes derived from the two cell lines (C2 and C3) were observed, probably indicating towards differential effects of cells towards the drug. However, these changes were only observed in doses far beyond the ETPC range. Such discrepant outcomes may be due to the unique pharmacodynamic/pharmacokinetic characteristics of amiodarone whereby it is metabolized slowly and has a long retention period that warrants a very low prescribed dose. Nevertheless, the most significant cFPD effects of amiodarone were observed at 1 µM which is consistent to its hERG blocking ability (IC50 1 µM).

Verapamil (Class IV) did not demonstrate cFPD prolongation but shortening as clinically. However, these shortenings were observed beyond its ETPC unbound range (25-81 nM). However, the fact that verapamil (Class IV) did not prolong the cFPD is of interest since there is a close margin between its ETPC unbound (25-81 nM) and hERG IC₅₀ (140-830 nM) that would suggest otherwise. It is important to note that for this reason, verapamil has been a classical example for "false positive" in the conventional hERG assay (Braam *et al.,* (2010). Apart from verapamil (Class IV), sotalol is also of interest in this context as hERG assay would not capture QT effects of sotalol at ETPC unbound (1.8-14 µM) as its hERG IC₅₀ is 74 µM. It is noteworthy that effects of both these drugs were captured using the present hiPSC system, which could have been mispredicted with the conventional hERG assay.

In conclusion, the results show that hiPSC-CM effectively capture the electrophysiological properties and pharmaceutical responses of drugs that match clinical observations on QT modulations and arrhythmia. The results also show that while cFPD prolongation could be used as readouts for QT prolongation, FPmin levels provide additional indications on drugs interactions with voltage-gated sodium channel. Furthermore, the results show that different cell line-derived cardiomyocytes may show variations, which may reflect differential drug response within patient cohorts. Moreover, the reduced BRV of hiPSC-CMs from individual with genetically predisposed arrhythmia further supported the feasibility of using such hiPSC-CMs to prognosticate cardiac arrhythmia.

Importantly, the examined drugs demonstrated dose-response curve that correlated significantly with therapeutic plasma concentrations achieved clinically. Moreover, drug-induced arrhythmia with tachycardia and bigeminy-like waveforms was demonstrated in selective agents at higher doses that are consistent with clinical observations. These results suggest that hiPSC-CMs may be useful for early evaluation of cardioactive drugs and enhance personalized medicine.

### References

Bode and Olejniczak (2002) Fundamental and Clinical Pharmacology 16(2):105-118
Braam et al., (2010) Stem Cell Research (2010) 4:107-116
Cavero and Crumb (2005) Expert Opinion on Drug Safety 4(3):509-30
Kodama et al., (1997), Cardiovascular Research 35:13-29
Lombardi et al., (2001) Cardiovascular research, 50:210-217.
Mehta et al., (2010) Cell Biology International, 34:1021-1031.
Mehta et al., (2011) Cardiovascular research 91:577-589.
Redfern et al., (2003) Cardiovascular Research 58: 32-45.
Yookoo et al., (2009) Biochem Biophys Res Commun. 387:482-8;
Zwi et al., (2009) Circulation 120:1513-1523.

## Claims

1. An in vitro method for selecting a dosage or dosage range of an agent as a drug candidate customised to an individual subject, comprising the steps of:
(i) contacting at least one induced pluripotent stem cell (iPSC)-derived cardiomyocyte-like cell derived from said subject with the agent at more than two different dosages; and
(ii) determining the effect of the agent based on changes in at least one electrophysiological property of the iPSC-derived cardiomyocyte-like cell contacted with different dosages of the agent at the different dosages, compared to a control comprising at least one iPSC-derived cardiomyocyte absent the agent to give a dose response curve;
and selecting a suitable dosage or dosage range exerting a desired effect on the induced pluripotent stem cell (iPSC)-derived cardiomyocyte-like cell(s) from the subject.

2. The method according to claim 1, wherein the electrophysiological property comprises the field potential duration (FPD), minimum field potential (FPmin), conduction velocity, beating frequency and/or regularity of beating rhythm.

3. The method according to claim 1 or 2, wherein the method comprises determining if the agent causes an increase, decrease or no changes in at least one of field potential duration (FPD), minimum field potential (FPmin), conduction velocity and beating frequency, compared to said control, and/or wherein the method comprises determining if the agent causes a change or no changes in regularity of beating rhythm compared to said control.

4. The method according to claim 1 or 2, wherein the method is for screening for an agent capable of exerting any one of the following effects on cardiomyocytes:
(i) increasing the field potential duration (FPD) compared to said control;
(ii) decreasing the field potential duration (FPD) compared to said control;
(iii) increasing the minimum field potential (FPmin) compared to said control;
(iv) decreasing the minimum field potential (FPmin) compared to said control;
(v) increasing the beating frequency compared to said control;
(vi) decreasing the beating frequency compared to said control;
(vii) increasing conduction velocity compared to said control
(viii) decreasing conduction velocity compared to said control; or
(ix) disrupting the regularity of rhythmic waveform compared to said control.

5. The method according to any one of the preceding claims, comprising using a multielectrode array or a patch-clamp to measure the electrophysiological properties in step (ii).

6. The method according to any one of the preceding claims, wherein the method is for screening for a cardiovascular agent, an agent affecting cardiac function and/or an agent for ameliorating, controlling, eliminating, preventing, reducing and/or treating a cardiac condition.

7. The method according to any one of the preceding claims, wherein the method is for screening for anti-arrhythmic agents; and/or
assessing the cardiotoxicity of the agent over a dosage range.

8. Use of induced pluripotent stem cell (iPSC)-derived cardiomyocyte-like cell(s) in a method according to any one of the preceding claims.

## Patentansprüche

1. In-Vitro-Verfahren zum Auswählen einer Dosierung oder eines Dosierungsbereichs eines Wirkmittels als an ein einzelnes Subjekt angepasster Wirkstoffkandidat, umfassend die Schritte:
(i) Inkontaktbringen mindestens einer aus induzierten pluripotenten Stammzellen (IPSC)-gewonnene Kardiomyozyten-ähnliche Zelle, welche vom Subjekt mit dem Wirkmittel bei mehr als zwei unterschiedlichen Dosierungen; und
(ii) Bestimmen der Wirkung des Wirkmittels auf der Basis von Änderungen in mindestens einer elektrophysiologischen Eigenschaft der aus IPSC gewonnenen Kardiomyozyten-ähnlichen Zelle, welche mit unterschiedlichen Dosierungen des Wirkmittels bei den verschiedenen Dosierungen in Kontakt gebracht wird, im Vergleich zu einer Kontrolle, welche mindestens einen aus IPSC gewonnenen Kardiomyozyten und keinem Wirkmittel umfasst, um eine Dosenreaktionskurve zu erhalten;
und Auswählen einer geeigneten Dosierung oder eines geeigneten Dosierungsbereichs, welche eine gewünschte Wirkung auf die aus induzierten pluripotenten Stammzellen (IPSC)-gewonnenen Kardiomyozyten-ähnlichen Zelle(n) vom Subjekt ausüben.

2. Verfahren nach Anspruch 1, wobei die elektrophysiologische Eigenschaft die Feldpotentialdauer (FPD), das Mindestfeldpotential (FPmin), die Leitgeschwindigkeit, die Schlagfrequenz und/oder die Regelmäßigkeit des Schlagrhythmus umfasst.

3. Verfahren nach Anspruch 1 oder 2, wobei das Verfahren das Bestimmen umfasst, ob das Wirkmittel eine Erhöhung, eine Reduktion oder keine Änderung in mindestens eines der Feldpotentialdauer (FPD), des Mindestfeldpotentials (FPmin), der Leitgeschwindigkeit und der Schlagfrequenz im Vergleich zu der Kontrolle bewirkt, und/oder wobei das Verfahren das Bestimmen umfasst, ob das Wirkmittel eine Änderung oder keine Änderung in der Regelmäßigkeit des Schlagrhythmus im Vergleich zur Kontrolle bewirkt.

4. Verfahren nach Anspruch 1 oder 2, wobei das Verfahren zum Screening eines Wirkmittels bestimmt ist, welches in der Lage ist, irgendeine der folgenden Wirkungen auf Kardiomyozyten auszuüben:
(i) Erhöhen der Feldpotentialdauer (FPD), im Vergleich zur Kontrolle;
(ii) Reduzieren der Feldpotentialdauer (FPD) im Vergleich zur Kontrolle;
(iii) Erhöhen des Mindestfeldpotentials (FPmin) im Vergleich zur Kontrolle;
(iv) Reduzieren des Mindestfeldpotentials (FPmin) im Vergleich zur Kontrolle;
(v) Erhöhen der Schlagfrequenz im Vergleich zur Kontrolle;
(vi) Reduzieren der Schlagfrequenz im Vergleich zur Kontrolle;
(vii) Erhöhen der Leitgeschwindigkeit im Vergleich zur Kontrolle;
(viii) Reduzieren der Leitgeschwindigkeit im Vergleich zur Kontrolle; oder
(ix) Unterbrechen der Regelmäßigkeit der rhythmischen Wellenform im Vergleich zur Kontrolle.

5. Verfahren nach einem der vorhergehenden Ansprüche, umfassend das Verwenden einer Mehrelektrodenanordnung oder einer Patch-Klemme um die elektrophysiologischen Eigenschaften im Schritt (ii) zu messen.

6. Verfahren nach einem der vorhergehenden Ansprüche, wobei das Verfahren zum Screening eines kardiovaskularen Wirkmittels, eines Wirkmittels, welches die Herzfunktion und/oder ein Wirkmittel zum Verbessern, Steuern, Entfernen, Verhindern, Reduzieren und/oder Behandeln einer Herzkrankheit bestimmt ist.

7. Verfahren nach einem der vorhergehenden Ansprüche, wobei das Verfahren zum Screening von anti-arrhythmischen Wirkmitteln; und/oder
zum Bewerten der Kardiotoxizität des Wirkmittels in einem Dosierbereich bestimmt ist.

8. Verwendung von aus induzierten pluripotenten Stammzellen (IPSC)-gewonnenen Kardiomyozyten-ähnlichen Zelle(n) in einem Verfahren nach einem der vorhergehenden Ansprüche.

## Revendications

1. Procédé in vitro pour sélectionner une posologie ou une plage de posologies d'un agent tel qu'un candidat de médicament qui est personnalisé pour un sujet individuel, comprenant les étapes constituées par :
(i) la mise en contact d'au moins une cellule du type cardiomyocyte dérivée à partir de cellules souches pluripotentes induites (iPSC) qui est dérivée à partir dudit sujet avec l'agent selon plus de deux posologies différentes ; et
(ii) la détermination de l'effet de l'agent sur la base de variations d'au moins une propriété électrophysiologique de la cellule du type cardiomyocyte dérivée à partir d'iPSC mise en contact avec des posologies différentes de l'agent selon les posologies différentes, comparé à un contrôle qui comprend au moins un cardiomyocyte dérivé à partir d'iPSC en l'absence de l'agent de manière à obtenir une courbe de réponse à la posologie ;
et la sélection d'une posologie appropriée ou d'une plage de posologies appropriées exerçant un effet souhaité sur la/les cellule(s) du type cardiomyocyte dérivée(s) à partir de cellules souches pluripotentes induites (iPSC) à partir du sujet.

2. Procédé selon la revendication 1, dans lequel la propriété électrophysiologique comprend la durée de potentiel de champ (FPD), le potentiel de champ minimum (FPmin), la vitesse de conduction, la fréquence de battement et/ou la régularité du rythme de battement.

3. Procédé selon la revendication 1 ou 2, dans lequel le procédé comprend la détermination de si l'agent génère une augmentation, une diminution ou une absence de variation d'au moins une propriété prise parmi la durée de potentiel de champ (FPD), le potentiel de champ minimum (FPmin), la vitesse de conduction et la fréquence de battement, en comparaison audit contrôle, et/ou dans lequel le procédé comprend la détermination de si l'agent génère une variation ou une absence de variation de la régularité du rythme de battement en comparaison audit contrôle.

4. Procédé selon la revendication 1 ou 2, dans lequel le procédé a pour objet de présélectionner un agent disposant de la capacité d'exercer l'un quelconque des effets qui suivent sur les cardiomyocytes :
(i) l'augmentation de la durée de potentiel de champ (FPD) en comparaison audit contrôle ;
(ii) la diminution de la durée de potentiel de champ (FPD) en comparaison audit contrôle ;
(iii) l'augmentation du potentiel de champ minimum (FPmin) en comparaison audit contrôle ;
(iv) la diminution du potentiel de champ minimum (FPmin) en comparaison audit contrôle ;
(v) l'augmentation de la fréquence de battement en comparaison audit contrôle ;
(vi) la diminution de la fréquence de battement en comparaison audit contrôle ;
(vii) l'augmentation de la vitesse de conduction en comparaison audit contrôle ;
(viii) la diminution de la vitesse de conduction en comparaison audit contrôle ; ou
(ix) la perturbation de la régularité de la forme d'onde rythmique en comparaison audit contrôle.

5. Procédé selon l'une quelconque des revendications qui précèdent, comprenant l'utilisation d'un réseau à multiples électrodes ou d'un patch-clamp pour mesurer les propriétés électrophysiologiques selon l'étape (ii).

6. Procédé selon l'une quelconque des revendications qui précèdent, dans lequel le procédé a pour objet de présélectionner un agent cardiovasculaire, un agent affectant la fonction cardiaque et/ou un agent pour améliorer, contrôler, éliminer, empêcher, réduire et/ou traiter une condition cardiaque.

7. Procédé selon l'une quelconque des revendications qui précèdent, dans lequel le procédé a pour objet de présélectionner des agents antiarythmiques ; et/ou
d'évaluer la cardiotoxicité de l'agent sur une plage de posologies.

8. Utilisation d'une/de cellule(s) du type cardiomyocyte dérivée(s) à partir de cellules souches pluripotentes induites (iPSC) selon un procédé selon l'une quelconque des revendications qui précèdent.
